(19) Europäisches Patentamt
European Patent Office
Office européen des brevets

(11) Veröffentlichungsnummer: 0 385 084 A2

(12) EUROPÄISCHE PATENTANMELDUNG

(21) Anmeldenummer: 90101155.1

(22) Anmeldetag: 20.01.90

(51) Int. Cl.5: C07D 233/90, A01N 43/50, C07D 401/04, A01N 43/40

(30) Priorität: 27.01.89 DE 3902439

(43) Veröffentlichungstag der Anmeldung: 05.09.90 Patentblatt 90/36

(84) Benannte Vertragsstaaten: BE CH DE FR GB IT LI NL

(71) Anmelder: BASF Aktiengesellschaft
Carl-Bosch-Strasse 38
D-6700 Ludwigshafen(DE)

(72) Erfinder: Wriede, Ulrich, Dr.
Birkenstrasse 7
D-6704 Mutterstadt(DE)
Erfinder: Hamprecht, Gerhard, Dr.
Rote-Turm-Strasse 28
D-6940 Weinheim(DE)
Erfinder: Koehler, Hermann, Dr.
In den Obstgaerten 7
D-6719 Bobenheim(DE)
Erfinder: Wuerzer, Bruno, Dr.
Ruedigerstrasse 13
D-6701 Otterstadt(DE)

# (54) Pflanzenschützende Mittel auf Basis von 1-Aryl- bzw. 1-Hetarylimidazolcarbonsäureestern.

(57) 1-Aryl- und 1-Hetarylimidazolcarbonsäureester der allgemeinen Formeln Ia und Ib

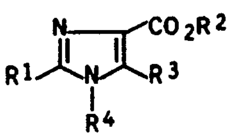

Ia

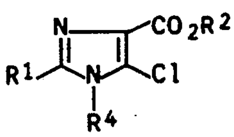

Ib

in denen die Substituenten folgende Bedeutung haben:
$R^1$ Halogen oder ggf. substituiertes $C_1$-$C_4$-Alkyl;
$R^2$ ggf. substituiertes $C_1$-$C_6$-Alkyl; $C_3$-$C_6$-Alkenyl, $C_3$-$C_6$-Alkinyl oder $C_3$-$C_6$-Cycloalkyl;
$R^3$ Wasserstoff oder $C_1$-$C_4$-Alkyl;
$R^4$ ggf. substituiertes Phenyl oder 5- oder 6-gliedriges Heteroaryl.
Verfahren zur Herstellung und sie enthaltende Mittel.

EP 0 385 084 A2

## Pflanzenschützende Mittel auf Basis von 1-Aryl- bzw. 1-Hetarylimidazolcarbonsäureestern

Die vorliegende Erfindung betrifft 1-Aryl- und 1-Hetarylimidazolcarbonsäureester der allgemeinen Formeln Ia und Ib

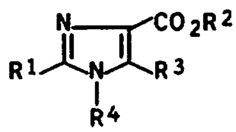

Ia

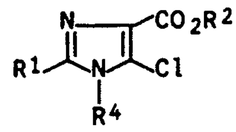

Ib

in denen die Substituenten folgende Bedeutung haben:
$R^1$ ein Halogenatom oder eine $C_1$-$C_4$-Alkylgruppe, welche ein bis drei Chloratome tragen kann,
$R^2$ eine $C_1$-$C_6$-Alkylgruppe, welche ein bis drei der folgenden Reste tragen kann: Halogen, $C_1$-$C_4$-Alkoxy und/oder $C_1$-$C_4$-Alkoxy-$C_1$-$C_4$-alkoxy, eine $C_3$-$C_6$-Alkenylgruppe, eine $C_3$-$C_6$-Alkinylgruppe oder eine $C_3$-$C_6$-Cycloalkylgruppe,
$R^3$ Wasserstoff oder eine $C_1$-$C_4$-Alkylgruppe,
$R^4$ ein Phenylring oder ein 5- oder 6-gliedriger Heteroaromat, enthaltend ein oder zwei Stickstoffatome und/oder ein Sauerstoff oder ein Schwefelatom im Ring, wobei diese aromatischen Ringe ein bis drei der folgenden Gruppen tragen können: Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, $C_1$-$C_4$-Alkylsulfinyl, $C_1$-$C_4$-Alkylsulfonyl, $C_1$-$C_4$-Alkylcarbonyl, $C_1$-$C_4$-Alkoxycarbonyl, Carboxyl, Nitro und/oder Cyano.

Außerdem betrifft die Erfindung Verfahren zur Herstellung der Verbindungen Ia und Ib sowie herbizide Mittel, die 2-(4-Heteroaryloxy)- oder 2-(4-Aryloxy)-phenoxyessig- oder -propionsäurederivate und/oder Cyclohexenonderivate als herbizide Wirkstoffe und Aryl- bzw. Hetarylimidazolcarbonsäurederivate als Antidots enthalten, sowie Verfahren zur selektiven Bekämpfung von unerwünschtem Pflanzenwuchs mit diesen herbiziden Mitteln.

Aus der EP-A 174 562 sind 1-Aryltriazolcarbonsäurederivate mit pflanzenschützender Wirkung bekannt. Daneben sind 1-Arylimidazolcarbonsäureester mit wachstumsregulierenden Eigenschaften (EP-A 243 615, EP-A 264 577) und 2-Halogenimidazolcarbonsäureester mit herbizider Wirkung beschrieben (EP-A 127 446; EP-A 180 787; US-A 4.711.962; US-A 4.591.377 und US-A 4.578.106).

Herbizide Wirkstoffe aus der Gruppe der 2-(4-Heteroaryloxy)- oder 2-(4-Aryloxy)-phenoxyessigsäurederivate der Formel IV,

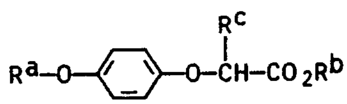

IV

in der die Substituenten folgende Bedeutung haben:
$R^a$ eine Phenylgruppe, eine Pyridylgruppe, eine Benzoxazylgruppe, eine Benzthiazylgruppe oder eine Benzpyrazinylgruppe, wobei diese aromatischen Ringsysteme bis zu zwei der folgenden Reste tragen können: Halogen, $C_1$-$C_4$-Alkyl, $C_1$-$C_4$-Halogenalkyl und/oder $C_1$-$C_4$-Halogenphenoxy
$R^b$ Wasserstoff, eine $C_1$-$C_5$-Alkylgruppe, eine $C_3$-$C_5$-Alkylideniminogruppe, eine $C_3$-$C_5$-Alkylideniminooxy-$C_2$-$C_3$-alkylgruppe, oder das Äquivalent eines pflanzenverträglichen Kations und
$R^c$ Wasserstoff oder eine Methylgruppe,
dienen der Bekämpfung von unerwünschten Pflanzen aus der Familie der Gramineen (z.B. DE-A 22 23 894, DE-A 24 33 067, DE-A 25 76 251, DE-A 30 04 770, BE-A 868 875 und BE-A 858 618). Die Verträglichkeit dieser Substanzen für Kulturpflanzen variiert jedoch zwischen kommerziell acceptabel und unverträglich, je nach Substituenten und Aufwandmenge.

Ebenso verhält es sich mit den Cyclohexenonderivaten der Formel V,

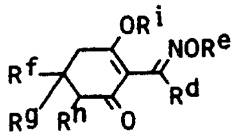

V

in welcher die Substituenten folgende Bedeutung haben:
$R^d$ eine $C_1$-$C_4$-Alkylgruppe;
$R^e$ eine $C_1$-$C_4$-Alkylgruppe, eine $C_3$-$C_4$-Alkenylgruppe, eine $C_3$-$C_4$-Alkinylgruppe, eine $C_3$-$C_4$-Halogenalkenylgruppe oder eine Thenylgruppe, welche durch ein Halogenatom substituiert sein kann;
$R^f$ eine $C_1$-$C_4$-Alkylgruppe, welche einfach durch $C_1$-$C_4$-Alkylthio oder $C_1$-$C_4$-Alkoxy substituiert sein kann; ein 5- oder 6-gliedriges gesättigtes oder einfach ungesättigtes Ringsystem, welches neben Kohlenstoffgliedern ein Sauerstoff-, ein Schwefelatom oder eine Sulfoxid- oder Sulfongruppe enthalten kann, und dieser Ring bis zu drei der folgenden Reste tragen kann: Hydroxy, Halogen, $C_1$-$C_4$-Alkyl, $C_1$-$C_4$-Halogenalkyl, $C_1$-$C_4$-Alkoxy und/oder $C_1$-$C_4$-Alkylthio;
einen 10-gliedrigen gesättigten oder einfach ungesättigten Heterocyclus, welcher zwei Sauerstoffatome oder Schwefelatome enthält und durch bis zu drei $C_1$-$C_4$-Alkylgruppen und/oder Methoxygruppen substituiert sein kann;
eine Phenylgruppe, eine Pyridylgruppe oder eine Isoxazolylgruppe, wobei diese Gruppen bis zu drei der folgenden Reste tragen können: $C_1$-$C_4$-Alkyl, $C_1$-$C_4$-Halogenalkyl, $C_1$-$C_4$-Alkoxy, $C_1$-$C_4$-Alkylthio, $C_3$-$C_6$-Alkenyloxy, $C_3$-$C_6$-Alkinyloxy, $C_1$-$C_4$-Alkoxy-$C_1$-$C_3$-alkyl, $C_1$-$C_4$-Dialkoxy-$C_1$-$C_3$-alkyl, Formyl, Halogen und/oder Benzoylamino;
$R^g$ Wasserstoff, Hydroxy oder, wenn $R^f$ die Bedeutung $C_1$-$C_6$-Alkylgruppe hat, eine $C_1$-$C_6$-Alkylgruppe;
$R^h$ Wasserstoff, eine Cyanogruppe, ein Halogenatom oder eine $C_1$-$C_4$-Alkoxycarbonylgruppe und
$R^i$ Wasserstoff oder das Äquivalent eines umweltverträglichen Kations.

Sie sind in der Literatur ebenfalls als Herbizide beschrieben (z.B. EP-A 228 598, EP-A 230 235, EP-A 238 021, US-A 4 432 786, DE-A 24 39 104) und dienen vorwiegend zur Bekämpfung unerwünschter Gräser in dicotylen Kulturen und in Gräsern, die nicht zur Familie der Gramineen zählen. Je nach Struktur der Substituenten und Dosis der Anwendung können Verbindungen aus dieser Gruppe auch zur selektiven Bekämpfung von unerwünschten Gräsern in Gramineen Kulturen wie Weizen und Reis eingesetzt werden.

Der Erfindung lagen daher Verbindungen als Aufgabe zugrunde, welche die Nachteile bei der Verwendung der obengenannten Herbizide der Formeln IV und V beheben oder zumindest stark verringern. Solche Verbindungen werden auch als "Antidote" oder "Safener" bezeichnet.

Entsprechend der Aufgabe wurden die eingangs definierten 1-Aryl- und 1-Hetarylimidazoncarbonsäureester Ia und Ib gefunden. Weiterhin wurden Verfahren zur Herstellung dieser Verbindungen Ia und Ib sowie zur gemeinsamen Anwendung dieser Verbindungen mit den Herbiziden IV und V zur Beeinflussung unerwünschten Pflanzenwachstums gefunden. Außerdem betrifft die Erfindung Mittel, welche die Verbindungen Ia und/oder Ib sowie Herbizide des Typs IV oder V enthalten, wobei es unerheblich ist, ob der herbizide Wirkstoff und die antidotische Verbindung gemeinsam oder getrennt formuliert und ausgebracht werden bzw. bei getrennter Ausbringung, in welcher Reihenfolge herbizider Wirkstoff und Antidot appliziert werden.

Die erfindungsgemäßen Verbindungen der Formeln Ia und Ib sind auf verschiedenen Wegen zugänglich.

So erhält man die Verbindungen Ia beispielsweise, indem man einen Imidazolcarbonsäureester der Formel II in an sich bekannter Weise in einem inerten organischen Lösungsmittel in Gegenwart einer Base mit einem Aryl- oder Hetarylderivat III zu Ia umsetzt.

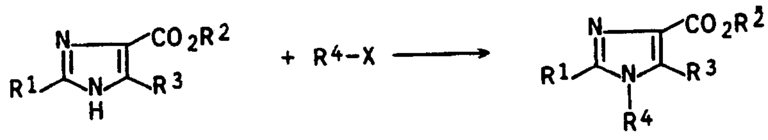

II III Ia

X in der Formel III bedeutet dabei ein Halogenatom wie Fluor, Chlor und Brom, vorzugsweise Fluor und Chlor.

Die Reaktion kann kontinuierlich oder diskontinuierlich, bei Normaldruck oder Drucken bis 30 bar, vorzugsweise 1 bis 10 bar, und Temperaturen von 20 °C bis 200 °C, vorzugsweise von 50 °C bis 170 °C, durchgeführt werden.

Als Lösungsmittel eignen sich beispielsweise n-Hexan, Dekalin, Toluol, Xylol, Chlorbenzol, Tetrahydrofuran, Dioxan, Dimethylformamid, Diethylformamid, Dimethylsulfoxid, Aceton und Ethanol. Bevorzugt werden Toluol und Dimethylformamid.

Als Basen kommen Natriumbicarbonat, Natriumcarbonat, Kaliumcarbonat, Natriumhydroxid, Kaliumhydroxid, Natriumhydrid, Natriummethylat, Natriumethylat, Kalium-tert.-butylat, Triethylamin, Diisopropylethylamin, N,N-Dimethylanilin, Pyridin, Isochinolin, N-Methylpyrolidin, N,N,N′,N′-Tetramethylethylendiamin oder 1,8-Diazabicyclo(5,4,0)undec-7-en in Betracht. Vorzugsweise dienen jedoch Natriumhydrid, Natriumethylat und Triethylamin als Base.

Die Herstellung der hierfür benötigten Imidazolcarbonsäureester ist u.a. in der ES-A 512 649 sowie in der EP-A 127 446 beschrieben.

Die Verbindungen Ib lassen sich aus den Aryl- bzw. Hetarylimidazolcarbonsäureestern Ia, in denen $R^3$ Wasserstoff bedeutet, im allgemeinen üblicher Weise durch Umsetzung mit einem Chlorierungsmittel herstellen.

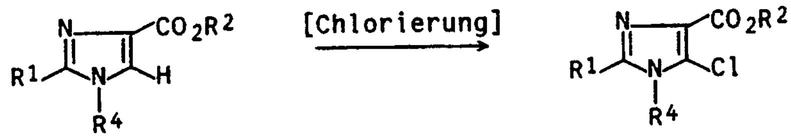


Ia ($R^3$ = H) Ib

Geeignete Chlorierungsmittel sind beispielsweise Phosphoroxytrichlorid, Sulfurylchlorid, Thionylchlorid, Phosgen, Phosphorpentachlorid, Phosphortrichlorid oder Schwefeltetrachlorid, wobei Sulfurylchlorid bevorzugt wird.

Die Reaktion wird üblicherweise in Gegenwart oder Abwesenheit eines inerten organischen Lösungsmittels wie Chlorbenzol und Dichlorbenzol kontinuierlich oder diskontinuierlich bei Temperaturen von 20 °C bis 200 °C, vorzugsweise 80 °C bis 150 °C, bei Normaldruck oder leichtem Überdruck (1-5 bar) durchgeführt.

Sofern man die Reaktion in Gegenwart eines der obengenannten Lösungsmittel durchführt, empfiehlt es sich, einen Radikalstarter wie Dibenzoylperoxid und Azobisisobutyrontril zur Beschleunigung der Reaktion mitzuverwenden.

In Hinsicht auf die bestimmungsgemäße Verwendung der Verbindungen Ia und Ib als pflanzenschützende Mittel kommen als Substituenten folgende Reste in Betracht:

$R^1$ Alkylgruppen wie Methyl, Ethyl, Propyl, iso-Propyl, n-Butyl, sec-Butyl, iso-Butyl und tert.-Butyl, vorzugsweise Methyl und Ethyl;

Halogenalkylgruppen wie Chlormethyl, Dichlormethyl, Trichlormethyl, 1,1-Dichlorethyl, 1-Chlorethyl, vorzugsweise Chlormethyl, Dichlormethyl und Trichlormethyl;

Halogenatome wie Fluor, Chlor, Brom und Jod, bevorzugt Chlor und Brom;

$R^2$ Alkylgruppen wie unter R1 genannt sowie n-Pentyl, 2-Methylbutyl, 3-Methylbutyl, 2,2, -Dimethylpropyl, n-Hexyl, 2-Methylpentyl, 3-Methylpentyl, 4-Methylpentyl, 2,2-Dimethylbutyl, 3,3-Dimethylbutyl, 2,3-Dimethylbutyl sowie 2-Ethylbutyl, vorzugsweise Methyl und Ethyl;

Alkenylgruppen wie Allyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1,2-Dimethyl-2-propenyl, 1,1-Dimethyl-2-propenyl, 1-Methyl-3-butenyl, 1-Ethyl-2-propenyl, 2-Hexenyl, 1-Methyl-2-pentenyl, 1-Ethyl-2-butenyl und 2-Ethyl-2-butenyl, vorzugsweise Allyl, 2-Butenyl und 1-Methyl-2-propenyl;

Alkinylgruppen wie 2-Propinyl, 2-Butinyl, 3-Butinyl, 1-Methyl-2-propinyl, 1-Methyl-2-butinyl, 1-Ethyl-2-propinyl, 2-Pentinyl, 3-Pentinyl, 4-Pentinyl und 1-Methyl-2-pentinyl, vorzugsweise 2-Propinyl, 1-Methyl-2-propinyl und 2-Butinyl;

Cycloalkylgruppen wie Cyclopropan, Cyclobutan, Cyclopentan und Cyclohexan, vorzugsweise Cyclopropan, Cyclopentan und Cyclohexan;

Halogenalkylgruppen wie unter $R^1$ genannt sowie Fluormethyl, Difluormethyl, Trifluormethyl, 2-Fluorethyl, 2,2-Difluorethyl und 2,2,2-Trifluorethyl, vorzugsweise Difluormethyl, Trifluormethyl, Chlormethyl, Dichlormethyl, Trichlormethyl und 2-Chorethyl;

Alkoxyalkylgruppen wie 2-Methoxyethyl, 2-Ethoxyethyl, 2-Propoxyethyl, 3-Methoxypropyl und 3-Ethoxypropyl, vorzugsweise Methoxyethyl und 2-Ethoxyethyl;

Alkoxyalkoxyalkylgruppen wie 2-Methoxymethoxyethyl, 2-Ethoxymethoxyethyl und 2-Ethoxyethoxyethyl.

$R^3$ Wasserstoff oder eine Alkylgruppe wie unter $R^1$ genant, vorzugsweise Wasserstoff, Methyl und Ethyl;

$R^4$ ein Phenylring oder
ein 5- oder 6-gliedriger Heteroaromat wie Pyrrol, Pyrazol, Imidazol, Furan, Thiophen, Oxazol, Thiazol, Isoxazol, Isothiazol, Pyridin, Pyridazin, Pyrimidin und Pyrazin, vorzugsweise Pyridin und Pyrimidin;

Bevorzugte Substituenten von $R^4$ sind
Alkylgruppen wie unter $R^1$ genannt, insbesondere Methyl, Ethyl und iso-Propyl;
Halogenalkylgruppen wie unter $R^2$ genannt, insbesondere Trifluormethyl und Difluormethyl;
Alkoxygruppen wie Methoxy, Ethoxy, Propoxy, 1-Methylethoxy, Butoxy, 1-Methyloxy, 2-Methyloxy oder 1,1-Dimethylethoxy, insbesondere Methoxy, Ethoxy und 2-Propoxy;
Halogenalkoxygruppen wie Trifluormethoxy, Trichlormethoxy, Fluormethoxy, 2,2,2-Trifluorethoxy, 1,2,2-Trifluormethoxy oder 1,1,2,2-Tetrafluorethoxy, insbesondere Trifluormethoxy und 2,2,2-Trifluorethoxy;
Alkylthiogruppen wie Methylthio, Ethylthio, Propylthio, 2-Propylthio, Butylthio, 1-Methylpropylthio, 2-Methylpropylthio oder 1,1-Dimethylethylthio, insbesondere Methylthio und Ethylthio;
Halogenalkylthiogruppen wie Trifluormethylthio und Trichlormethylthio,
Alkylsulfinylgruppen wie Methylsulfinyl und Ethylsulfinyl,
Alkylsulfonylgruppen wie Methylsulfonyl und Ethylsulfonyl,
Alkylcarbonylgruppen wie Acetyl, Ethylcarbonyl, Propylcarbonyl, 1-Methylethylcarbonyl, Butylcarbonyl, 1-Methylpropylcarbonyl, 2-Methylpropylcarbonyl oder 1,1-Dimethylethylcarbonyl, besonders Acetyl, 1-Methylpropylcarbonyl und 1,1-Dimethylethylcarbonyl;
Alkoxycarbonylgruppen wie Methoxycarbonyl, Ethoxycarbonyl, Propyloxycarbonyl und 1-Methylethoxycarbonyl;
Halogenatome wie Fluor, Chlor und Brom, insbesondere Fluor und Chlor sowie Carboxyl-, Nitro- und Cyanogruppen.

Besonders bevorzugt sind Verbindungen Ia und Ib, in denen der Rest $R^4$ unsubstituiert oder ein- oder zweifach substituiert ist.

Spezielle Beispiele für herbizide (Heteroaryloxy)- bzw. Aryloxy-phenoxyessigsäurederivate der Formel IV, deren Kulturpflanzenverträglichkeit durch Aryl- bzw. Hetarylimidazolcarbonsäureester der Formeln Ia und Ib verbessert werden kann, sind in der folgenden Tabelle A aufgeführt:

Tabelle A

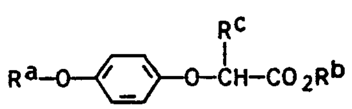

IV

| Nr. | $R^a$ | $R^b$ | $R^c$ | Lit |
|---|---|---|---|---|
| IV.1 | 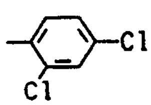 | $CH_3$ | $CH_3$ | DE-A 22 23 894 |
| IV.2 | 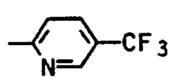 | n-$C_4H_9$ | $CH_3$ | BE-A 868 875 |
| IV.3 | 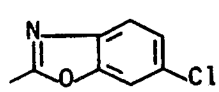 | $C_2H_5$ | $CH_3$ | BE-A 858 618 |
| IV.4 | 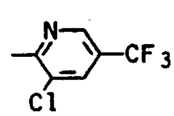 | $CH_3$ | $CH_3$ | BE-A 868 875 |
| IV.5 | 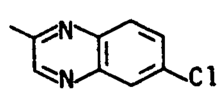 | $C_2H_5$ | $CH_3$ | DE-A 30 04 770 |

Spezielle Beispiele für Cyclohexenone der Formel V, deren Kulturpflanzenverträglichkeit durch 1-Aryl- und 1-Hetarylimidazolcarbonsäureester der Formeln Ia und Ib verbessert werden kann, sind in der folgenden Tabelle B aufgeführt.

Tabelle B

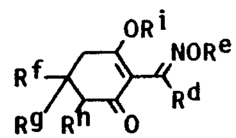 V

| Nr. | $R^d$ | $R^e$ | $R^f$ | $R^g$ | $R^h$ | $R^i$ | Literatur |
|---|---|---|---|---|---|---|---|
| V.1 | $C_3H_7$ | $CH_2CH=CH_2$ | $CH_3$ | $CH_3$ | $CO_2CH_3$ | Na | DE-A 2 439 104 |
| V.2 | $C_3H_7$ | $CH_2CH_3$ | $CH_2CH(CH_3)SCH_2CH_3$ | H | H | H | DE-A 2 822 304 |
| V.3 | $C_2H_5$ | $CH_2CH=CHCl$ | $CH_2CH(CH_3)SCH_2CH_3$ | H | H | H | US-A 4 440 566 |
| V.4 | $C_3H_7$ | $CH_2CH=CHCl$ | $CH_2CH(CH_3)SCH_2CH_3$ | H | H | H | US-A 4 440 566 |
| V.5 | $C_3H_7$ | $C_2H_5$ | 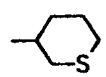 | H | H | H | EP-A 71 707 |
| V.6 | $C_2H_5$ | $C_2H_5$ | 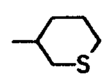 | H | H | H | EP-A 71 707 |

| Nr. | $R^d$ | $R^e$ | $R^f$ | $R^g$ | $R^h$ | $R^i$ | Literatur |
|---|---|---|---|---|---|---|---|
| V.7 | $CH_3$ | $CH_2CH=CHCH_3$ | 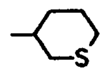 | H | H | H | EP-A 71 707 |
| V.8 | $C_3H_7$ | $C_2H_5$ | 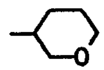 | H | H | H | EP-A 71 707 |
| V.9 | $C_2H_5$ | $CH_2CH=CHCl$ | 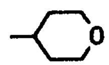 | H | H | H | EP-A 142 741 |
| V.10 | $C_3H_7$ | $C_2H_5$ | 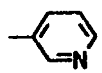 | H | H | H | EP-A 66 195 |
| V.11 | $C_2H_5$ | $C_2H_5$ | 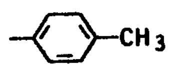 | H | H | H | DE-A 24 39 104 |
| V.12 | $C_2H_5$ | $CH_2CH=CHCH_3$ | 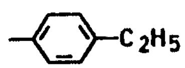 | H | H | H | DE-A 38 08 072 |
| V.13 | $C_2H_5$ | $C_2H_5$ | 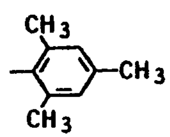 | H | H | H | EP-A 880 301 |
| V.14 | $C_3H_7$ | $CH_2CH=CHCl$ | 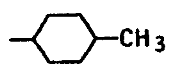 | H | H | H | EP-A 88 299 |
| V.15 | $C_3H_7$ | $CH_2CH=CHCH_3$ | 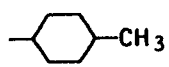 | H | H | H | EP-A 88 299 |

| Nr. | $R^d$ | $R^e$ | $R^f$ | $R^g$ | $R^h$ | $R^i$ | Literatur |
|---|---|---|---|---|---|---|---|
| V.16 | $C_2H_5$ | $CH_2CH=CHCH_3$ | 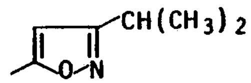 | H | H | H | EP-A 238 021 |
| V.17 | $C_3H_7$ | $CH_2CH=CHCH_3$ | 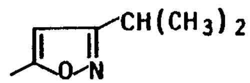 | H | H | H | EP-A 238 021 |
| V.18 | $C_2H_5$ | $CH_2CH=CHCl$ | 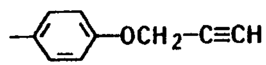 | H | H | H | EP-A 137 174 |
| V.19 | $C_3H_7$ | $C_2H_5$ | 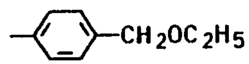 | H | H | H | EP-A 2 137 200 |
| V.20 | $C_3H_7$ | $C_2H_5$ | 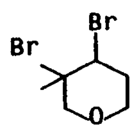 | H | H | H | EP-A 230 235 |
| V.21 | $C_3H_7$ | $CH_2CH=CHCl$ | 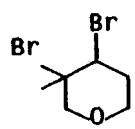 | H | H | H | EP-A 230 235 |

| Nr. | $R^d$ | $R^e$ | $R^f$ | $R^g$ | $R^h$ | $R^i$ | Literatur |
|---|---|---|---|---|---|---|---|
| V.22 | $C_3H_7$ | $CH_2CH=CHCl$ | 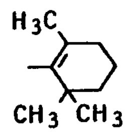 | H | H | H | EP-A 46 860 |
| V.23 | $C_3H_7$ | $C_2H_5$ | 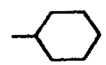 | H | H | H | JP-A 540 191 945 |
| V.24 | $C_3H_7$ | $C_2H_5$ | 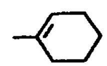 | H | H | H | EP-A 46 860 |
| V.25 | $CH_3$ | $CH_2CH=CHCl$ | 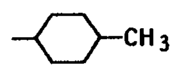 | H | H | H | EP-A 88 299 |
| V.26 | $C_3H_7$ | $C_2H_5$ | 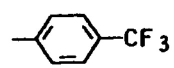 | H | H | K | EP-A 137 174 |
| V.27 | $C_2H_5$ | $CH_2CH=CHCl$ | 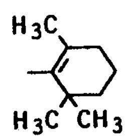 | H | H | H | EP-A 46 860 |

Herbizide Wirkstoffe und antidotisch wirkende Verbindungen können gemeinsam oder getrennt nach dem Auflaufen auf die Blätter und Sprosse der Kulturpflanzen und der unerwünschten Gräser ausgebracht werden. Bevorzugt wird das antidotisch wirkende Mittel gleichzeitig mit dem herbiziden Wirkstoff ausgebracht. Auch eine getrennt Ausbringung, wobei das Antidot zuerst und anschließend der herbizide Wirkstoff auf das Feld gebracht werden, ist möglich. Herbizider Wirkstoff und Antidot können hierbei als Spritzmittel in suspendierbarer, emulgierbarer oder löslicher Form gemeinsam oder getrennt formuliert vorliegen.

Möglich ist auch eine Behandlung der Kulturpflanzensamen mit dem Antidot vor der Aussaat. Der herbizide Wirkstoff wird dann allein in der üblichen Weise appliziert.

Für herbizide (Hetero)aryloxyphenoxyessig- oder -propionsäurederivate der Formel IV werden unterschiedliche Mengen einer antidotisch wirkenden Verbindung benötigt, wenn das Herbizid in verschiedenen Kulturen eingesetzt wird. Die Mengenverhältnisse sind in breiten Bereichen variabel. Sie sind ebenfalls abhängig von der Struktur der (Hetero)aryloxyphenoxyessig- oder -propionsäurederivate der Formel IV und der jeweiligen Zielkultur. Geeignete Anteilverhältnisse herbizider Wirkstoff : antidotisch wirkende Verbindung liegen zwischen 1:4 bis 1:0,01; vorzugsweise zwischen 1:4 bis 1:0,1 Gew.-Teile.

Für das gleiche Cyclohexenonderivat V werden unterschiedliche Mengen einer antidotisch wirkenden Verbindung benötigt, wenn das Cyclohexenonderivat in verschiedenen Kulturen eingesetzt wird. Die Mengenverhältnisse, in denen ein Cyclohexenonderivat und ein Aryl- bzw. Heteroarylimidazolcarbonsäureester der Formel Ia und/oder Ib eingesetzt werden, sind in breiten Bereichen variabel. Sie sind abhängig von der Struktur des Cyclohexenonderivats, des Aryl- bzw. Heteroarylimidazolcarbonsäurresters der Formel Ia und/oder Ib und der jeweiligen Kultur. Geeignete Anteilverhältnisse herbizider Wirkstoff : antidotisch wirkende Verbindung liegen zwischen 1:4 bis 1:0,01; vorzugsweise 1:4 bis 1:0,25 Gew.-Teile.

Die neuen herbiziden Mittel können neben dem Aryl- bzw. Hetarylimidazolcarbonsäureester der Formel Ia und/oder Ib als Antidot und dem Herbizid aus der Gruppe der (Hetero)aryloxyphenoxyessig- oder -propionsäuren IV bzw. der Cyclohexenone V weitere herbizide und wachstumsregulierende Wirkstoffe andere inerte zusätze enthalten, wobei der antagonistische Effekt erhalten bleibt.

Die erfindungsgemäßen Mittel bzw. bei getrennter Ausbringung die herbiziden Wirkstoffe oder das Antidot werden beispielsweise in Form von direkt versprühbaren Lösungen, Pulvern, Suspensionen, auch hochprozentigen wäßrigen, öligen oder sonstigen Suspensionen, oder Dispersionen, Emulsionen, Öldispersionen, Pasten, Stäubemitteln, Streumitteln oder Granulaten durch Versprühen, Vernebeln, Verstäuben, Verstreuen oder Gießen angewendet. Die Anwendungsformen richten sich ganz nach den Verwendungszwecken.

Zur Herstellung von direkt versprühbaren Lösungen, Emulsionen, Pasten und Ölsdispersionen kommen Mineralölfraktionen von mittlerem bis hohem Siedepunkt, wie Kerosin oder Dieselöl, ferner Kohlenteeröle, sowie Öle pflanzlichen oder tierischen Ursprungs, aliphatische, cyclische oder aromatische Kohlenwasserstoffe, z.B. Benzol, Toluol, Xylol, Paraffin, Tetrahydronaphthalin, alkylierte Naphthaline oder deren Derivate, z.B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z.B. Dimethylformamid, Dimethylsulfoxid, N-Methylpyrrolidon und Wasser, in Betracht.

Wäßrige Anwendungsformen können aus Emulsionskonzentraten, Pasten oder netzbaren Pulvern (Spritzpulvern), Öldispersionen durch zusatz von Wasser bereitet werden. Zur Herstellung von Emulsionen, Pasten oder Öldispersionen können herbizider Wirkstoff und/oder Antidot als solche oder in einem Öl oder Lösungsmittel gelöst, mittels Netz-, Haft-, Dispergier- oder Emulgiermittel in Wasser homogenisiert werden. Es können aber auch aus herbizidem Wirkstoff und/oder Antidot Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatierten Fettalkoholglykolethern, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol-, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin-Sulfitablaugen und Methylcellulose in Betracht.

Pulver, Streu- und Stäubmittel können durch Mischen oder gemeinsames Vermahlen von herbizidem Wirkstoff und/oder Antidot mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Silicagel, Kieselsäuren, Kieselgele, Silikate, Talkum, Kaolin, Attaclay, Kalkstein, Kreide, Talkum, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehle, Baumrinden-, Holz- und Nußschalenmehl, Cellulosepulver und andere feste Trägerstoffe.

Herstellungsbeispiele

Die in den nachstehenden Beispielen wiedergegebenen Vorschriften wurden unter entsprechender Abwandlung der Ausgangsverbindungen zur Gewinnung weiterer Verbindungen der Formeln Ia und Ib benutzt; die erhaltenen Verbindungen sind in den nachfolgenden Tabellen mit physikalischen Angaben aufgeführt; Verbindungen ohne diese Angaben lassen sich aus den entsprechenden Stoffen in analoger Weise aufbauen. Sie lassen aufgrund ihrer nahen strukturellen Beziehungen zu den hergestellten und untersuchten Verbindungen eine gleichartige Wirkung erwarten.

Beispiel 1

1-(2,4-Dinitrophenyl)-2-methylimidazol-4-carbonsäure-ethylester

14,5 g (0,078 mol) 2,4-Dinitrofluorbenzol, 12 g (0,078 mol) 2-Methylimidazol-4-carbonsäureethylester und 500 ml Toluol wurden mit 16 ml Triethylamin versetzt wonach die Mischung 18 Stunden unter Rückfluß erhitzt wurde. Anschließend wurde das Lösungsmittel bei vermindertem Druck abdestilliert und der Rückstand mit Pentan/Essigester als Laufmittel chromatographiert.

Ausbeute: 19,5 g (81 % d. Th.) Fp.: 150-152°C
(Wirkstoffbeispiel 1.015)

Beispiel 2

1-(2,4-Dinitrophenyl)-2-brom-5-methyl-4-imidazolcarbonsäure-isopropylester

Zu 5 g (0,02 mol) 2-Brom-5-methyl-4-imidazolcarbonsäure-isopropylester, 3,9 g 1-Fluor-2,4-dinitrobenzol und 200 ml Toluol wurden tropfenweise 4,2 ml Triethylamin gegeben, wonach die Mischung 16 Stunden zum Rückfluß erhitzt wurde. Zur Aufarbeitung wurde die Toluollösung mit Wasser gewaschen, getrocknet und eingeengt. Der Rückstand wurde mit Diethylether verrührt und der so erhaltene Feststoff isoliert.

Ausbeute: 5,7 g (69 %) Fp.: 161-162°C
(Wirkstoffbeispiel 1.025)

Beispiel 3

1-(2,4-Dibromphenyl)-2-methylimidazol-4-carbonsäure-methylester

Zu 10,9 g (0,078 mol) 2-Methylimidazol-4-carbonsäure-methylester in 100 ml Dimethylformamid gab man 14,1 g 30 % Natriummethylatlösung und entfernte das entstehende Methanol bei vermindertem Druck vollständig. Die verbleibende Dimethylformamid-Lösung wurde mit 38,1 g (0,15 mol) 1-Fluor-2,4-dibrombenzol versetzt und 2 Stunden unter Rückfluß gekocht. Anschließend wurde Waser zugesetzt, mit Essigsäureethylester extrahiert und die Essigsäureethylesterphase mit Wasser gewaschen. Aus der organischen Phase erhielt man nach Entfernen des Lösungsmittels einen Rückstand, der mit Diisopropylether verrührt kristallisierte.

Ausbeute: 10,8 g (36,7 %) Fp.: 162-163°C
(Wirkstoffbeispiel 1.059)

Beispiel 4

1-(2,4-Dinitrophenyl)-2-methyl-5-chlorimidazol-4-carbonsäure-ethylester und 1-(2,4-Dinitrophenyl)-2-trichlormethyl-5-chlor-imidazol-4-carbon-säureethylester

9,8 g (0,03 mol) 1-(2,4-Dinitrophenyl)-2-methylimidazol-4-carbonsäure-ethylester in 200 ml Chlorbenzol wurden mit 16,5 g (0,1225 mol) Sulforylchlorid und 0,2 g Dibenzoylperoxid versetzt und für 1 1/2 Stunden auf 100°C erhitzt. Nach Beendigung der Gasentwicklung wurde zur Trockene eingeengt, der Rückstand mit verdünntem Ammoniakwasser auf pH 7 eingestellt, mit Methylenchlorid extrahiert, die organische Phase mit Natriumsulfat getrocknet, eingeengt und chromatographiert. Ausbeute: 1,8 g (16 %), Fp.: 149-150°C (Wirkstoffbeispiel 2.002) und
4,7 g (43 % d. Th.) Fp.: 181-182°C (Wirkstoffbeispiel 2.001).

Tabelle 1

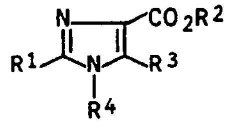

| Nr. | $R^1$ | $R^2$ | $R^3$ | $R^4$ | Fp(°C) ; NMR(ppm) |
|---|---|---|---|---|---|
| 1.001 | $CH_3$ | $CH_3$ | H | Phenyl | |
| 1.002 | $CH_3$ | $CH_2CH_3$ | H | Phenyl | |
| 1.003 | Cl | $CH_3$ | $CH_3$ | Phenyl | |
| 1.004 | Cl | $CH_2CH_3$ | $CH_3$ | Phenyl | |
| 1.005 | Cl | $CH(CH_3)_2$ | $CH_3$ | Phenyl | |
| 1.006 | Br | $CH_3$ | $CH_3$ | Phenyl | |
| 1.007 | Br | $CH_2CH_3$ | $CH_3$ | Phenyl | |
| 1.008 | $CH_3$ | $CH_3$ | H | 4-Nitro-phenyl | |
| 1.009 | $CH_3$ | $CH_2CH_3$ | H | 4-Nitro-phenyl | |
| 1.010 | $CH_3$ | $CH(CH_3)_2$ | H | 4-Nitro-phenyl | |
| 1.011 | $CH_3$ | $CH_2CH{=}CH_2$ | H | 4-Nitro-phenyl | |
| 1.012 | Cl | $CH_2CH_3$ | $CH_3$ | 4-Nitro-phenyl | |
| 1.013 | Br | $CH_2CH_3$ | $CH_3$ | 4-Nitro-phenyl | |
| 1.014 | $CH_3$ | $CH_3$ | H | 2,4-Dinitro-phenyl | |
| 1.015 | $CH_3$ | $CH_2CH_3$ | H | 2,4-Dinitro-phenyl | 150-152 |
| 1.016 | $CH_3$ | $CH(CH_3)_2$ | H | 2,4-Dinitro-phenyl | |
| 1.017 | $CH_3$ | $CH_2CH_2CH_3$ | H | 2,4-Dinitro-phenyl | |
| 1.018 | $CH_3$ | $CH_2CH{=}CH_2$ | H | 2,4-Dinitro-phenyl | |
| 1.019 | $CH_3$ | $CH_2C{\equiv}CH$ | H | 2,4-Dinitro-phenyl | |
| 1.020 | Cl | $CH_3$ | $CH_3$ | 2,4-Dinitro-phenyl | |
| 1.021 | Cl | $CH_2CH_3$ | $CH_3$ | 2,4-Dinitro-phenyl | |
| 1.022 | Cl | $CH(CH_3)_2$ | $CH_3$ | 2,4-Dinitro-phenyl | |
| 1.023 | Br | $CH_3$ | $CH_3$ | 2,4-Dinitro-phenyl | |
| 1.024 | Br | $CH_2CH_3$ | $CH_3$ | 2,4-Dinitro-phenyl | 148-150 |
| 1.025 | Br | $CH(CH_3)_2$ | $CH_3$ | 2,4-Dinitro-phenyl | 161-162 |
| 1.026 | $CH_3$ | $CH_3$ | H | 2-Nitro-phenyl | |
| 1.027 | $CH_3$ | $CH_2CH_3$ | H | 2-Nitro-phenyl | |
| 1.028 | Cl | $CH_2CH_3$ | $CH_3$ | 2-Nitro-phenyl | |
| 1.029 | Br | $CH_2CH_3$ | $CH_3$ | 2-Nitro-phenyl | |
| 1.030 | $CH_3$ | $CH_3$ | H | 4-Chlor-phenyl | |
| 1.031 | $CH_3$ | $CH_2CH_3$ | H | 4-Chlor-phenyl | |
| 1.032 | Cl | $CH_3$ | $CH_3$ | 4-Chlor-phenyl | |
| 1.033 | Cl | $CH_2CH_3$ | $CH_3$ | 4-Chlor-phenyl | |
| 1.034 | Br | $CH_3$ | $CH_3$ | 4-Chlor-phenyl | |
| 1.035 | Br | $CH_2CH_3$ | $CH_3$ | 4-Chlor-phenyl | |
| 1.036 | $CH_3$ | $CH_3$ | H | 2-Chlor-phenyl | |
| 1.037 | $CH_3$ | $CH_2CH_3$ | H | 2-Chlor-phenyl | |

Tabelle 1 (Fortsetzung)

| Nr. | $R^1$ | $R^2$ | $R^3$ | $R^4$ | Fp(°C); NMR(ppm) |
|---|---|---|---|---|---|
| 1.038 | $CH_3$ | $CH_3$ | H | 2,4-Dichlor-phenyl | 162-163 |
| 1.039 | $CH_3$ | $CH_2CH_3$ | H | 2,4-Dichlor-phenyl | 121-122 |
| 1.040 | $CH_3$ | $CH_2CH_2CH_3$ | H | 2,4-Dichlor-phenyl | |
| 1.041 | $CH_3$ | $CH(CH_3)_2$ | H | 2,4-Dichlor-phenyl | |
| 1.042 | Cl | $CH_3$ | $CH_3$ | 2,4-Dichlor-phenyl | |
| 1.043 | Cl | $CH_2CH_3$ | $CH_3$ | 2,4-Dichlor-phenyl | |
| 1.044 | Cl | $CH(CH_3)_2$ | $CH_3$ | 2,4-Dichlor-phenyl | |
| 1.045 | Cl | Cyclopentyl | $CH_3$ | 2,4-Dichlor-phenyl | |
| 1.046 | Cl | Cyclohexyl | $CH_3$ | 2,4-Dichlor-phenyl | |
| 1.047 | Br | $CH_3$ | $CH_3$ | 2,4-Dichlor-phenyl | |
| 1.048 | Br | $CH_2CH_3$ | $CH_3$ | 2,4-Dichlor-phenyl | |
| 1.049 | Br | $CH_2CH_2CH_3$ | $CH_3$ | 2,4-Dichlor-phenyl | |
| 1.050 | Br | $CH(CH_3)_2$ | $CH_3$ | 2,4-Dichlor-phenyl | |
| 1.051 | $CH_3$ | $CH_3$ | H | 4-Brom-phenyl | |
| 1.052 | $CH_3$ | $CH_2CH_3$ | H | 4-Brom-phenyl | |
| 1.053 | Cl | $CH_2CH_3$ | $CH_3$ | 4-Brom-phenyl | |
| 1.054 | Br | $CH_2CH_3$ | $CH_3$ | 4-Brom-phenyl | |
| 1.055 | $CH_3$ | $CH_3$ | H | 2-Brom-phenyl | |
| 1.056 | $CH_3$ | $CH_2CH_3$ | H | 2-Brom-phenyl | |
| 1.057 | Cl | $CH_2CH_3$ | $CH_3$ | 2-Brom-phenyl | |
| 1.058 | Br | $CH_2CH_3$ | $CH_3$ | 2-Brom-phenyl | |
| 1.059 | $CH_3$ | $CH_3$ | H | 2,4-Dibrom-phenyl | 162-163 |
| 1.060 | $CH_3$ | $CH_2CH_3$ | H | 2,4-Dibrom-phenyl | |
| 1.061 | $CH_3$ | $CH_2CH_2CH_3$ | H | 2,4-Dibrom-phenyl | |
| 1.062 | $CH_3$ | $CH(CH_3)_2$ | H | 2,4-Dibrom-phenyl | |
| 1.063 | Cl | $CH_2CH_3$ | $CH_3$ | 2,4-Dibrom-phenyl | |
| 1.064 | Cl | $CH(CH_3)_2$ | $CH_3$ | 2,4-Dibrom-phenyl | |
| 1.065 | Br | $CH_2CH_3$ | $CH_3$ | 2,4-Dibrom-phenyl | |
| 1.066 | Br | $CH(CH_3)_2$ | $CH_3$ | 2,4-Dibrom-phenyl | |
| 1.067 | $CH_3$ | $CH_3$ | H | 2,4,6-Trichlor-phenyl | |
| 1.068 | $CH_3$ | $CH_2CH_3$ | H | 2,4,6-Trichlor-phenyl | |
| 1.069 | $CH_3$ | $CH_2CH_2CH_3$ | H | 2,4,6-Trichlor-phenyl | |
| 1.070 | Cl | $CH_2CH_3$ | $CH_3$ | 2,4,6-Trichlor-phenyl | |
| 1.071 | Br | $CH_2CH_3$ | $CH_3$ | 2,4,6-Trichlor-phenyl | |
| 1.072 | Br | $CH_2CH_2CH_3$ | $CH_3$ | 2,4,6-Trichlor-phenyl | |
| 1.073 | $CH_3$ | $CH_3$ | H | 4-Cyano-phenyl | |
| 1.074 | $CH_3$ | $CH_2CH_3$ | H | 4-Cyano-phenyl | |
| 1.075 | $CH_3$ | $CH_2CH_2CH_3$ | H | 4-Cyano-phenyl | |
| 1.076 | $CH_3$ | $CH(CH_3)_2$ | H | 4-Cyano-phenyl | |
| 1.077 | Cl | $CH_2CH_3$ | $CH_3$ | 4-Cyano-phenyl | |
| 1.078 | Cl | $CH(CH_3)_2$ | $CH_3$ | 4-Cyano-phenyl | |

Tabelle 1 (Fortsetzung)

| Nr. | $R^1$ | $R^2$ | $R^3$ | $R^4$ | Fp(°C); NMR(ppm) |
|---|---|---|---|---|---|
| 1.079 | Br | $CH_2CH_3$ | $CH_3$ | 4-Cyano-phenyl | |
| 1.080 | Br | $CH(CH_3)_2$ | $CH_3$ | 4-Cyano-phenyl | |
| 1.081 | $CH_3$ | $CH_3$ | H | 2,4-Dicyano-phenyl | |
| 1.082 | $CH_3$ | $CH_2CH_3$ | H | 2,4-Dicyano-phenyl | |
| 1.083 | Cl | $CH_2CH_3$ | $CH_3$ | 2,4-Dicyano-phenyl | |
| 1.084 | Br | $CH_2CH_3$ | $CH_3$ | 2,4-Dicyano-phenyl | |
| 1.085 | $CH_3$ | $CH_3$ | H | 4-Trifluormethyl-phenyl | |
| 1.086 | $CH_3$ | $CH_2CH_3$ | H | 4-Trifluormethyl-phenyl | |
| 1.087 | $CH_3$ | $CH_2CH_2CH_3$ | H | 4-Trifluormethyl-phenyl | |
| 1.088 | Cl | $CH_2CH_3$ | $CH_3$ | 4-Trifluormethyl-phenyl | |
| 1.089 | Br | $CH_2CH_3$ | $CH_3$ | 4-Trifluormethyl-phenyl | |
| 1.090 | $CH_3$ | $CH_3$ | H | 4-Chlor-2-nitro-phenyl | |
| 1.091 | $CH_3$ | $CH_2CH_3$ | H | 4-Chlor-2-nitro-phenyl | |
| 1.092 | Cl | $CH_2CH_3$ | $CH_3$ | 4-Chlor-2-nitro-phenyl | |
| 1.093 | Br | $CH_2CH_3$ | $CH_3$ | 4-Chlor-2-nitro-phenyl | |
| 1.094 | $CH_3$ | $CH_3$ | H | 4-Chlor-2-fluor-phenyl | |
| 1.095 | $CH_3$ | $CH_2CH_3$ | H | 4-Chlor-2-fluor-phenyl | |
| 1.096 | Cl | $CH_2CH_3$ | $CH_3$ | 4-Chlor-2-fluor-phenyl | |
| 1.097 | Br | $CH_2CH_3$ | $CH_3$ | 4-Chlor-2-fluor-phenyl | |
| 1.098 | $CH_3$ | $CH_3$ | H | 4-Chlor-2-cyano-phenyl | |
| 1.099 | $CH_3$ | $CH_2CH_3$ | H | 4-Chlor-2-cyano-phenyl | |
| 1.100 | Cl | $CH_2CH_3$ | $CH_3$ | 4-Chlor-2-cyano-phenyl | |
| 1.101 | Br | $CH_2CH_3$ | $CH_3$ | 4-Chlor-2-cyano-phenyl | |
| 1.102 | $CH_3$ | $CH_3$ | H | 4-Chlor-2-trifluor-methyl-phenyl | |
| 1.103 | $CH_3$ | $CH_2CH_3$ | H | 4-Chlor-2-trifluor-methyl-phenyl | |
| 1.104 | Cl | $CH_2CH_3$ | $CH_3$ | 4-Chlor-2-trifluor-methyl-phenyl | |
| 1.105 | Br | $CH_2CH_3$ | $CH_3$ | 4-Chlor-2-trifluor-methyl-phenyl | |
| 1.106 | $CH_3$ | $CH_3$ | H | 4-Chlor-2-methyl-phenyl | |
| 1.107 | $CH_3$ | $CH_2CH_3$ | H | 4-Chlor-2-methyl-phenyl | |
| 1.108 | Cl | $CH_2CH_3$ | $CH_3$ | 4-Chlor-2-methyl-phenyl | |
| 1.109 | Br | $CH_2CH_3$ | $CH_3$ | 4-Chlor-2-methyl-phenyl | |
| 1.110 | $CH_3$ | $CH_3$ | H | 4-Brom-2-nitro-phenyl | |
| 1.111 | $CH_3$ | $CH_2CH_3$ | H | 4-Brom-2-nitro-phenyl | |
| 1.112 | Cl | $CH_2CH_3$ | $CH_3$ | 4-Brom-2-nitro-phenyl | |
| 1.113 | Br | $CH_2CH_3$ | $CH_3$ | 4-Brom-2-nitro-phenyl | |
| 1.114 | $CH_3$ | $CH_3$ | H | 4-Brom-2-chlor-phenyl | |

Tabelle 1 (Fortsetzung)

| Nr. | $R^1$ | $R^2$ | $R^3$ | $R^4$ | Fp(°C); NMR(ppm) |
|---|---|---|---|---|---|
| 1.115 | $CH_3$ | $CH_2CH_3$ | H | 4-Brom-2-chlor-phenyl | |
| 1.116 | Cl | $CH_2CH_3$ | $CH_3$ | 4-Brom-2-chlor-phenyl | |
| 1.117 | Br | $CH_2CH_3$ | $CH_3$ | 4-Brom-2-chlor-phenyl | |
| 1.118 | $CH_3$ | $CH_3$ | H | 4-Brom-2-fluor-phenyl | |
| 1.119 | $CH_3$ | $CH_2CH_3$ | H | 4-Brom-2-fluor-phenyl | |
| 1.120 | Cl | $CH_2CH_3$ | $CH_3$ | 4-Brom-2-fluor-phenyl | |
| 1.121 | Br | $CH_2CH_3$ | $CH_3$ | 4-Brom-2-fluor-phenyl | |
| 1.122 | $CH_3$ | $CH_3$ | H | 4-Brom-2-cyano-phenyl | |
| 1.123 | $CH_3$ | $CH_2CH_3$ | H | 4-Brom-2-cyano-phenyl | |
| 1.124 | Cl | $CH_2CH_3$ | $CH_3$ | 4-Brom-2-cyano-phenyl | |
| 1.125 | Br | $CH_2CH_3$ | $CH_3$ | 4-Brom-2-cyano-phenyl | |
| 1.126 | $CH_3$ | $CH_3$ | H | 4-Brom-2-trifluor-methyl-phenyl | |
| 1.127 | $CH_3$ | $CH_2CH_3$ | H | 4-Brom-2-trifluor-methyl-phenyl | |
| 1.128 | Cl | $CH_2CH_3$ | $CH_3$ | 4-Brom-2-trifluor-methyl-phenyl | |
| 1.129 | Br | $CH_2CH_3$ | $CH_3$ | 4-Brom-2-trifluor-methyl-phenyl | |
| 1.130 | $CH_3$ | $CH_3$ | H | 4-Brom-2-methyl-phenyl | |
| 1.131 | $CH_3$ | $CH_2CH_3$ | H | 4-Brom-2-methyl-phenyl | |
| 1.132 | Cl | $CH_2CH_3$ | $CH_3$ | 4-Brom-2-methyl-phenyl | |
| 1.133 | Br | $CH_2CH_3$ | $CH_3$ | 4-Brom-2-methyl-phenyl | |
| 1.134 | $CH_3$ | $CH_3$ | H | 4-Cyano-2-nitro-phenyl | |
| 1.135 | $CH_3$ | $CH_2CH_3$ | H | 4-Cyano-2-nitro-phenyl | |
| 1.136 | Cl | $CH_2CH_3$ | $CH_3$ | 4-Cyano-2-nitro-phenyl | |
| 1.137 | Br | $CH_2CH_3$ | $CH_3$ | 4-Cyano-2-nitro-phenyl | |
| 1.138 | $CH_3$ | $CH_3$ | H | 4-Cyano-2-chlor-phenyl | |
| 1.139 | $CH_3$ | $CH_2CH_3$ | H | 4-Cyano-2-chlor-phenyl | |
| 1.140 | Cl | $CH_2CH_3$ | $CH_3$ | 4-Cyano-2-chlor-phenyl | |
| 1.141 | Br | $CH_2CH_3$ | $CH_3$ | 4-Cyano-2-chlor-phenyl | |
| 1.142 | $CH_3$ | $CH_3$ | H | 4-Cyano-2-fluor-phenyl | |
| 1.143 | $CH_3$ | $CH_2CH_3$ | H | 4-Cyano-2-fluor-phenyl | |
| 1.144 | Cl | $CH_2CH_3$ | $CH_3$ | 4-Cyano-2-fluor-phenyl | |
| 1.145 | Br | $CH_2CH_3$ | $CH_3$ | 4-Cyano-2-fluor-phenyl | |
| 1.146 | $CH_3$ | $CH_3$ | H | 4-Cyano-2-brom-phenyl | |
| 1.147 | $CH_3$ | $CH_2CH_3$ | H | 4-Cyano-2-brom-phenyl | 171-173 |
| 1.148 | $CH_3$ | $CH_2CH_2CH_3$ | H | 4-Cyano-2-brom-phenyl | |
| 1.149 | $CH_3$ | $CH(CH_3)_2$ | H | 4-Cyano-2-brom-phenyl | |
| 1.150 | Cl | $CH_2CH_3$ | $CH_3$ | 4-Cyano-2-brom-phenyl | |

Tabelle 1 (Fortsetzung)

| Nr. | $R^1$ | $R^2$ | $R^3$ | $R^4$ | Fp(°C); NMR(ppm) |
|---|---|---|---|---|---|
| 1.151 | Cl | $CH(CH_3)_2$ | $CH_3$ | 4-Cyano-2-brom-phenyl | |
| 1.152 | Cl | Cyclopentyl | $CH_3$ | 4-Cyano-2-brom-phenyl | |
| 1.153 | Cl | Cyclohexyl | $CH_3$ | 4-Cyano-2-brom-phenyl | |
| 1.154 | Br | $CH_2CH_3$ | $CH_3$ | 4-Cyano-2-brom-phenyl | |
| 1.155 | $CH_3$ | $CH_3$ | H | 4-Cyano-2-methyl-phenyl | |
| 1.156 | $CH_3$ | $CH_2CH_3$ | H | 4-Cyano-2-methyl-phenyl | |
| 1.157 | Cl | $CH_2CH_3$ | $CH_3$ | 4-Cyano-2-methyl-phenyl | |
| 1.158 | Br | $CH_2CH_3$ | $CH_3$ | 4-Cyano-2-methyl-phenyl | |
| 1.159 | $CH_3$ | $CH_3$ | H | 4-Cyano-2-trifluor-methyl-phenyl | |
| 1.160 | $CH_3$ | $CH_2CH_3$ | H | 4-Cyano-2-trifluor-methyl-phenyl | |
| 1.161 | Cl | $CH_2CH_3$ | $CH_3$ | 4-Cyano-2-trifluor-methyl-phenyl | |
| 1.162 | Br | $CH_2CH_3$ | $CH_3$ | 4-Cyano-2-trifluor-methyl-phenyl | |
| 1.163 | $CH_3$ | $CH_3$ | H | 4-Nitro-2-chlor-phenyl | |
| 1.164 | $CH_3$ | $CH_2CH_3$ | H | 4-Nitro-2-chlor-phenyl | 128-129 |
| 1.165 | Cl | $CH_2CH_3$ | $CH_3$ | 4-Nitro-2-chlor-phenyl | |
| 1.166 | Br | $CH_2CH_3$ | $CH_3$ | 4-Nitro-2-chlor-phenyl | |
| 1.167 | $CH_3$ | $CH_3$ | H | 4-Nitro-2-brom-phenyl | |
| 1.168 | $CH_3$ | $CH_2CH_3$ | H | 4-Nitro-2-brom-phenyl | |
| 1.169 | Cl | $CH_2CH_3$ | $CH_3$ | 4-Nitro-2-brom-phenyl | |
| 1.170 | Br | $CH_2CH_3$ | $CH_3$ | 4-Nitro-2-brom-phenyl | |
| 1.171 | $CH_3$ | $CH_3$ | H | 4-Nitro-2-cyano-phenyl | |
| 1.172 | $CH_3$ | $CH_2CH_3$ | H | 4-Nitro-2-cyano-phenyl | |
| 1.173 | Cl | $CH_2CH_3$ | $CH_3$ | 4-Nitro-2-cyano-phenyl | |
| 1.174 | Br | $CH_2CH_3$ | $CH_3$ | 4-Nitro-2-cyano-phenyl | |
| 1.175 | $CH_3$ | $CH_3$ | H | 4-Trifluormethyl-2-chlor-phenyl | |
| 1.176 | $CH_3$ | $CH_2CH_3$ | H | 4-Trifluormethyl-2-chlor-phenyl | |
| 1.177 | Cl | $CH_2CH_3$ | $CH_3$ | 4-Trifluormetyhl-2-chlor-phenyl | |
| 1.178 | Br | $CH_2CH_3$ | $CH_3$ | 4-Trifluormethyl-2-chlor-phenyl | |
| 1.179 | $CH_3$ | $CH_3$ | H | 4-Trifluormethyl-2-brom-phenyl | |

Tabelle 1 (Fortsetzung)

| Nr. | $R^1$ | $R^2$ | $R^3$ | $R^4$ | Fp(°C); NMR(ppm) |
|---|---|---|---|---|---|
| 1.180 | $CH_3$ | $CH_2CH_3$ | H | 4-Trifluormethyl-2-brom-phenyl | |
| 1.181 | Cl | $CH_2CH_3$ | $CH_3$ | 4-Trifluormethyl-2-brom-phenyl | |
| 1.182 | Br | $CH_2CH_3$ | $CH_3$ | 4-Trifluormethyl-2-brom-phenyl | |
| 1.183 | $CH_3$ | $CH_3$ | H | 4-Trifluormethyl-2-cyano-phenyl | |
| 1.184 | $CH_3$ | $CH_2CH_3$ | H | 4-Trifluormethyl-2-cyano-phenyl | |
| 1.185 | Cl | $CH_2CH_3$ | $CH_3$ | 4-Trifluormethyl-2-cyano-phenyl | |
| 1.186 | Br | $CH_2CH_3$ | $CH_3$ | 4-Trifluormethyl-2-cyano-phenyl | |
| 1.187 | $CH_3$ | $CH_3$ | H | 4-tert.-Butyl-2-chlor-phenyl | |
| 1.188 | $CH_3$ | $CH_2CH_3$ | H | 4-tert.-Butyl-2-chlor-phenyl | |
| 1.189 | Cl | $CH_2CH_3$ | $CH_3$ | 4-tert.-Butyl-2-chlor-phenyl | |
| 1.190 | Br | $CH_2CH_3$ | $CH_3$ | 4-tert.-Butyl-2-chlor-phenyl | |
| 1.191 | $CH_3$ | $CH_3$ | H | 4-tert.-Butyl-2-brom-phenyl | |
| 1.192 | $CH_3$ | $CH_2CH_3$ | H | 4-tert.-Butyl-2-brom-phenyl | |
| 1.193 | Cl | $CH_2CH_3$ | $CH_3$ | 4-tert.-Butyl-2-brom-phenyl | |
| 1.194 | Br | $CH_2CH_3$ | $CH_3$ | 4-tert.-Butyl-2-brom-phenyl | |
| 1.195 | $CH_3$ | $CH_3$ | H | 4-tert.-Butyl-2-cyano-phenyl | |
| 1.196 | $CH_3$ | $CH_2CH_3$ | H | 4-tert.-Butyl-2-cyano-phenyl | |
| 1.197 | Cl | $CH_2CH_3$ | $CH_3$ | 4-tert.-Butyl-2-cyano-phenyl | |
| 1.198 | Br | $CH_2CH_3$ | $CH_3$ | 4-tert.-Butyl-2-cyano-phenyl | |
| 1.199 | $CH_3$ | $CH_3$ | H | 2,6-Dichlor-4-trifluormethyl-phenyl | |

Tabelle 1 (Fortsetzung)

| Nr. | $R^1$ | $R^2$ | $R^3$ | $R^4$ | Fp(°C); NMR(ppm) |
|---|---|---|---|---|---|
| 1.200 | $CH_3$ | $CH_2CH_3$ | H | 2,6-Dichlor-4-trifluormethyl-phenyl | |
| 1.201 | Cl | $CH_2CH_3$ | $CH_3$ | 2,6-Dichlor-4-trifluormethyl-phenyl | |
| 1.202 | Br | $CH_2CH_3$ | $CH_3$ | 2,6-Dichlor-4-trifluormethyl-phenyl | |
| 1.203 | $CH_3$ | $CH_3$ | H | 2,6-Dichlor-4-nitro-phenyl | 205-207 |
| 1.204 | $CH_3$ | $CH_2CH_3$ | H | 2,6-Dichlor-4-nitro-phenyl | 149-151 |
| 1.205 | Cl | $CH_2CH_3$ | $CH_3$ | 2,6-Dichlor-4-nitro-phenyl | |
| 1.206 | Br | $CH_2CH_3$ | $CH_3$ | 2,6-Dichlor-4-nitro-phenyl | |
| 1.207 | $CH_3$ | $CH_3$ | H | 2,6-Dinitro-4-trifluormethyl-phenyl | |
| 1.208 | $CH_3$ | $CH_2CH_3$ | H | 2,6-Dinitro-4-trifluormethyl-phenyl | |
| 1.209 | Cl | $CH_2CH_3$ | $CH_3$ | 2,6-Dinitro-4-trifluormethyl-phenyl | |
| 1.210 | Br | $CH_2CH_3$ | $CH_3$ | 2,6-Dinitro-4-trifluormethyl-phenyl | |
| 1.211 | $CH_3$ | $CH_3$ | H | 2,4-Dinitro-6-cyano-phenyl | |
| 1.212 | $CH_3$ | $CH_2CH_3$ | H | 2,4-Dinitro-6-cyano-phenyl | |
| 1.213 | Cl | $CH_2CH_3$ | $CH_3$ | 2,4-Dinitro-6-cyano-phenyl | |
| 1.214 | Br | $CH_2CH_3$ | $CH_3$ | 2,4-Dinitro-6-cyano-phenyl | |
| 1.215 | $CH_3$ | $CH_3$ | H | 2,4-Dinitro-6-trifluormethyl-phenyl | |
| 1.216 | $CH_3$ | $CH_2CH_3$ | H | 2,4-Dinitro-6-trifluormethyl-phenyl | |
| 1.217 | Cl | $CH_2CH_3$ | $CH_3$ | 2,4-Dinitro-6-trifluormethyl-phenyl | |
| 1.218 | Br | $CH_2CH_3$ | $CH_3$ | 2,4-Dinitro-6-trifluormethyl-phenyl | |
| 1.219 | $CH_3$ | $CH_3$ | H | 2,4-Dinitro-6-carbethoxy-phenyl | |

Tabelle 1 (Fortsetzung)

| Nr. | $R^1$ | $R^2$ | $R^3$ | $R^4$ | Fp(°C); NMR(ppm) |
|---|---|---|---|---|---|
| 1.220 | $CH_3$ | $CH_2CH_3$ | H | 2,4-Dinitro-6-carb-ethoxy-phenyl | |
| 1.221 | Cl | $CH_2CH_3$ | $CH_3$ | 2,4-Dinitro-6-carb-ethoxy-phenyl | |
| 1.222 | Br | $CH_2CH_3$ | $CH_3$ | 2,4-Dinitro-6-carb-ethoxy-phenyl | |
| 1.223 | $CH_3$ | $CH_3$ | H | 2-Nitro-4-carb-ethoxy-phenyl | |
| 1.224 | $CH_3$ | $CH_2CH_3$ | H | 2-Nitro-4-carb-ethoxy-phenyl | |
| 1.225 | Cl | $CH_2CH_3$ | $CH_3$ | 2-Nitro-4-carb-ethoxy-phenyl | |
| 1.226 | Br | $CH_2CH_3$ | $CH_3$ | 2-Nitro-4-carb-ethoxy-phenyl | |
| 1.227 | $CH_3$ | $CH_3$ | H | 2-Nitro-4-acetyl-phenyl | |
| 1.228 | $CH_3$ | $CH_2CH_3$ | H | 2-Nitro-4-acetyl-phenyl | |
| 1.229 | Cl | $CH_2CH_3$ | $CH_3$ | 2-Nitro-4-acetyl-phenyl | |
| 1.230 | Br | $CH_2CH_3$ | $CH_3$ | 2-Nitro-4-acetyl-phenyl | |
| 1.231 | $CH_3$ | $CH_3$ | H | 2-Chlor-4-acetyl-phenyl | |
| 1.232 | $CH_3$ | $CH_2CH_3$ | H | 2-Chlor-4-acetyl-phenyl | |
| 1.233 | Cl | $CH_2CH_3$ | $CH_3$ | 2-Chlor-4-acetyl-phenyl | |
| 1.234 | Br | $CH_2CH_3$ | $CH_3$ | 2-Chlor-4-acetyl-phenyl | |
| 1.235 | $CH_3$ | $CH_3$ | H | 2,4-Dinitro-6-chlor-phenyl | |
| 1.236 | $CH_3$ | $CH_2CH_3$ | H | 2,4-Dinitro-6-chlor-phenyl | |
| 1.237 | Cl | $CH_2CH_3$ | $CH_3$ | 2,4-Dinitro-6-chlor-phenyl | |
| 1.238 | Br | $CH_2CH_3$ | $CH_3$ | 2,4-Dinitro-6-chlor-phenyl | |
| 1.239 | $CH_3$ | $CH_3$ | H | 2,6-Dinitro-4-methyl-sulfonyl-phenyl | |
| 1.240 | $CH_3$ | $CH_2CH_3$ | H | 2,6-Dinitro-4-methyl-sulfonyl-phenyl | |
| 1.241 | Cl | $CH_2CH_3$ | $CH_3$ | 2,6-Dinitro-4-methyl-sulfonyl-phenyl | |
| 1.242 | Br | $CH_2CH_3$ | $CH_3$ | 2,6-Dinitro-4-methyl-sulfonyl-phenyl | |
| 1.243 | $CH_3$ | $CH_3$ | H | 2,6-Dinitro-4-carbo-methoxy-phenyl | |
| 1.244 | $CH_3$ | $CH_2CH_3$ | H | 2,6-Dinitro-4-carbo-methoxy-phenyl | |

Tabelle 1 (Fortsetzung)

| Nr. | $R^1$ | $R^2$ | $R^3$ | $R^4$ | Fp(°C); NMR(ppm) |
|---|---|---|---|---|---|
| 1.245 | Cl | $CH_2CH_3$ | $CH_3$ | 2,6-Dinitro-4-carbo-methoxy-phenyl | |
| 1.246 | Br | $CH_2CH_3$ | $CH_3$ | 2,6-Dinitro-4-carbo-methoxy-phenyl | |
| 1.247 | $CH_3$ | $CH_3$ | H | 2,6-Dinitro-4-methyl-phenyl | |
| 1.248 | $CH_3$ | $CH_2CH_3$ | H | 2,6-Dinitro-4-methyl-phenyl | |
| 1.249 | Cl | $CH_2CH_3$ | $CH_3$ | 2,6-Dinitro-4-methyl-phenyl | |
| 1.250 | Br | $CH_2CH_3$ | $CH_3$ | 2,6-Dinitro-4-methyl-phenyl | |
| 1.251 | $CH_3$ | $CH_3$ | H | 3,5-Dichlor-4-pyridyl | |
| 1.252 | $CH_3$ | $CH_2CH_3$ | H | 3,5-Dichlor-4-pyridyl | |
| 1.253 | Cl | $CH_2CH_3$ | $CH_3$ | 3,5-Dichlor-4-pyridyl | |
| 1.254 | Br | $CH_2CH_3$ | $CH_3$ | 3,5-Dichlor-4-pyridyl | |
| 1.255 | $CH_3$ | $CH_3$ | H | 3-Chlor-5-trifluor-methyl-2-pyridyl | |
| 1.256 | $CH_3$ | $CH_2CH_3$ | H | 3-Chlor-5-trifluor-methyl-2-pyridyl | |
| 1.257 | Cl | $CH_2CH_3$ | $CH_3$ | 3-Chlor-5-trifluor-methyl-2-pyridyl | |
| 1.258 | Br | $CH_2CH_3$ | $CH_3$ | 3-Chlor-5-trifluor-methyl-2-pyridyl | |
| 1.259 | $CH_3$ | $CH_3$ | H | 3-Chlor-5-nitro-2-pyridyl | |
| 1.260 | $CH_3$ | $CH_2CH_3$ | H | 3-Chlor-5-nitro-2-pyridyl | |
| 1.261 | Cl | $CH_2CH_3$ | $CH_3$ | 3-Chlor-5-nitro-2-pyridyl | |
| 1.262 | Br | $CH_2CH_3$ | $CH_3$ | 3-Chlor-5-nitro-2-pyridyl | |
| 1.263 | $CH_3$ | $CH_3$ | H | 5-Chlor-3-trifluor-methyl-2-pyridyl | |
| 1.264 | $CH_3$ | $CH_2CH_3$ | H | 5-Chlor-3-trifluor-methyl-2-pyridyl | |
| 1.265 | Cl | $CH_2CH_3$ | $CH_3$ | 5-Chlor-3-trifluor-methyl-2-pyridyl | |
| 1.266 | Br | $CH_2CH_3$ | $CH_3$ | 5-Chlor-3-trifluor-methyl-2-pyridyl | |
| 1.267 | $CH_3$ | $CH_2CH_3$ | H | 3-Chlor-2-cyanophenyl | 136-139 |
| 1.268 | $CH_3$ | $CH_2CH_3$ | H | 3-Chlor-4-nitrophenyl | 102-103 |
| 1.269 | $CH_3$ | $CH_2CH_3$ | H | 2,6-Dibrom-4-nitrophenyl | 205-207 |

Tabelle 2

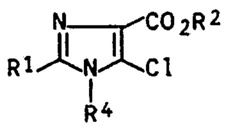

| Nr. | $R^1$ | $R^2$ | $R^4$ | Fp (°C) |
|---|---|---|---|---|
| 2.001 | $CH_3$ | $CH_2CH_3$ | 2,4-Dinitro-phenyl | 181-182 |
| 2.002 | $CCl_3$ | $CH_2CH_3$ | 2,4-Dinitro-phenyl | 149-151 |
| 2.003 | $CH_3$ | $CH_2CH_3$ | 2,4-Dichlor-phenyl | |
| 2.004 | $CCl_3$ | $CH_2CH_3$ | 2,4-Dichlor-phenyl | |
| 2.005 | $CH_3$ | $CH_2CH_3$ | 2,4-Dibrom-phenyl | |
| 2.006 | $CCl_3$ | $CH_2CH_3$ | 2,4-Dibrom-phenyl | |
| 2.007 | $CH_3$ | $CH_2CH_3$ | 2,4,6-Trichlor-phenyl | |
| 2.008 | $CCl_3$ | $CH_2CH_3$ | 2,4,6-Trichlor-phenyl | |
| 2.009 | $CH_3$ | $CH_2CH_3$ | 2,4-Dicyano-phenyl | |
| 2.010 | $CCl_3$ | $CH_2CH_3$ | 2,4-Dicyano-phenyl | |
| 2.011 | $CH_3$ | $CH_2CH_3$ | 4-Chlor-2-nitro-phenyl | |
| 2.012 | $CCl_3$ | $CH_2CH_3$ | 4-Chlor-2-nitro-phenyl | |
| 2.013 | $CH_3$ | $CH_2CH_3$ | 4-Chlor-2-fluor-phenyl | |
| 2.014 | $CCl_3$ | $CH_2CH_3$ | 4-Chlor-2-fluor-phenyl | |
| 2.015 | $CH_3$ | $CH_2CH_3$ | 4-Chlor-2-cyano-phenyl | |
| 2.016 | $CCl_3$ | $CH_2CH_3$ | 4-Chlor-2-cyano-phenyl | |
| 2.017 | $CH_3$ | $CH_2CH_3$ | 4-Chlor-2-trifluormethyl-phenyl | |
| 2.018 | $CCl_3$ | $CH_2CH_3$ | 4-Chlor-2-trifluormetyhl-phenyl | |
| 2.019 | $CH_3$ | $CH_2CH_3$ | 4-Brom-2-nitro-phenyl | |
| 2.020 | $CCl_3$ | $CH_2CH_3$ | 4-Brom-2-nitro-phenyl | |
| 2.021 | $CH_3$ | $CH_2CH_3$ | 4-Brom-2-chlor-phenyl | |
| 2.022 | $CCl_3$ | $CH_2CH_3$ | 4-Brom-2-chlor-phenyl | |
| 2.023 | $CH_3$ | $CH_2CH_3$ | 4-Brom-2-fluor-phenyl | |
| 2.024 | $CCl_3$ | $CH_2CH_3$ | 4-Brom-2-fluor-phenyl | |
| 2.025 | $CH_3$ | $CH_2CH_3$ | 4-Brom-2-cyano-phenyl | |
| 2.026 | $CCl_3$ | $CH_2CH_3$ | 4-Brom-2-cyano-phenyl | |
| 2.027 | $CH_3$ | $CH_2CH_3$ | 4-Brom-2-trifluormethyl-phenyl | |
| 2.028 | $CCl_3$ | $CH_2CH_3$ | 4-Brom-2-trifluormethyl-phenyl | |
| 2.029 | $CH_3$ | $CH_2CH_3$ | 4-Cyano-2-chlor-phenyl | |
| 2.030 | $CCl_3$ | $CH_2CH_3$ | 4-Cyano-2-chlor-phenyl | |
| 2.031 | $CH_3$ | $CH_2CH_3$ | 4-Cyano-2-fluor-phenyl | |
| 2.032 | $CCl_3$ | $CH_2CH_3$ | 4-Cyano-2-fluor-phenyl | |
| 2.033 | $CH_3$ | $CH_2CH_3$ | 4-Cyano-2-nitro-phenyl | |
| 2.034 | $CCl_3$ | $CH_2CH_3$ | 4-Cyano-2-nitro-phenyl | |

Tabelle 2 (Fortsetzung)

| Nr. | $R^1$ | $R^2$ | $R^4$ | Fp (°C) |
|---|---|---|---|---|
| 2.035 | $CH_3$ | $CH_2CH_3$ | 4-Cyano-2-brom-phenyl | |
| 2.036 | $CCl_3$ | $CH_2CH_3$ | 4-Cyano-2-brom-phenyl | |
| 2.037 | $CH_3$ | $CH_2CH_3$ | 4-Cyano-2-trifluormethyl-phenyl | |
| 2.038 | $CCl_3$ | $CH_2CH_3$ | 4-Cyano-2-trifluormethyl-phenyl | |
| 2.039 | $CH_3$ | $CH_2CH_3$ | 4-Nitro-2-chlor-phenyl | 119-120 |
| 2.040 | $CCl_3$ | $CH_2CH_3$ | 4-Nitro-2-chlor-phenyl | 157-158 |
| 2.041 | $CH_3$ | $CH_2CH_3$ | 4-Nitro-2-brom-phenyl | |
| 2.042 | $CCl_3$ | $CH_2CH_3$ | 4-Nitro-2-brom-phenyl | |
| 2.043 | $CH_3$ | $CH_2CH_3$ | 4-Nitro-2-cyano-phenyl | |
| 2.044 | $CCl_3$ | $CH_2CH_3$ | 4-Nitro-2-cyano-phenyl | |
| 2.045 | $CH_3$ | $CH_2CH_3$ | 4-Trifluormethyl-2-chlor-phenyl | |
| 2.046 | $CCl_3$ | $CH_2CH_3$ | 4-Trifluormethyl-2-chlor-phenyl | |
| 2.047 | $CH_3$ | $CH_2CH_3$ | 4-Trifluormethyl-2-brom-phenyl | |
| 2.048 | $CCl_3$ | $CH_2CH_3$ | 4-Trifluormethyl-2-brom-phenyl | |
| 2.049 | $CH_3$ | $CH_2CH_3$ | 4-Trifluormethyl-2-cyano-phenyl | |
| 2.050 | $CCl_3$ | $CH_2CH_3$ | 4-Trifluormethyl-2-cyano-phenyl | |
| 2.051 | $CH_3$ | $CH_2CH_3$ | 2,6-Dichlor-4-trifluormethyl-phenyl | |
| 2.052 | $CCl_3$ | $CH_2CH_3$ | 2,6-Dichlor-4-trifluormethyl-phenyl | |
| 2.053 | $CH_3$ | $CH_2CH_3$ | 2,6-Dinitro-4-trifluormethyl-phenyl | |
| 2.054 | $CCl_3$ | $CH_2CH_3$ | 2,6-Dinitro-4-trifluormethyl-phenyl | |
| 2.055 | $CH_3$ | $CH_2CH_3$ | 2,4-Dinitro-6-cyano-phenyl | |
| 2.056 | $CCl_3$ | $CH_2CH_3$ | 2,4-Dinitro-6-cyano-phenyl | |
| 2.057 | $CH_3$ | $CH_2CH_3$ | 2,4-Dinitro-6-trifluormethyl-phenyl | |
| 2.058 | $CCl_3$ | $CH_2CH_3$ | 2,4-Dinitro-6-trifluormethyl-phenyl | |
| 2.059 | $CH_3$ | $CH_2CH_3$ | 2,4-Dinitro-6-carbethoxy-phenyl | |
| 2.060 | $CCl_3$ | $CH_2CH_3$ | 2,4-Dinitro-6-carbethoxy-phenyl | |
| 2.061 | $CH_3$ | $CH_2CH_3$ | 2-Nitro-4-carbethoxy-phenyl | |
| 2.062 | $CCl_3$ | $CH_2CH_3$ | 2-Nitro-4-carbethoxy-phenyl | |
| 2.063 | $CH_3$ | $CH_2CH_3$ | 2,6-Dichlor-4-nitro-phenyl | |
| 2.064 | $CCl_3$ | $CH_2CH_3$ | 2,6-Dichlor-4-nitro-phenyl | 185-187 |

Anwendungsbeispiel

Der Einfluß verschiedener Vertreter der erfindungsgemäßen herbiziden Mittel bzw. Mittelkombinationen, bestehend aus Herbizid und antidotisch wirkender Verbindung, auf das Wachstum von erwünschten und unerwünschten Pflanzen im Vergleich zum herbiziden Wirkstoff allein wird durch die folgenden biologischen Beispiele aus Gewächshausversuchen belegt:

Zur Anzucht der Testpflanzen dienen Plastikblumentöpfe mit rund 300 $cm^3$ Inhalt und lehmiger Sand mit etwa 3,0 % Humus als Substrat. Die Samen der Testpflanzen werden nach Arten getrennt, flach eingesät und befeuchtet. Danach deckt man die Gefäße mit durchsichtigen Plastikhauben ab, bis die Samen gleichmäßig gekeimt und die Pflanzen angewachsen sind.

Für die Nachauflaufbehandlung zieht man die Testpflanzen je nach Wuchsform erst bis zu einer Wuchshöhe von 3 bis 20 cm an und behandelt sie dann. Die herbiziden Mittel werden hierbei in Wasser als Verteilungsmittel suspendiert und emulgiert und mittels fein verteilender Düsen gespritzt.

Als Cyclohexenonderivat V wurde in den biologischen Beispielen V.2 verwendet:

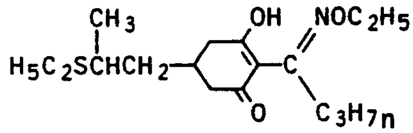

V.2

Die Formulierungen enthalten zwischen 0,1 und 95 Gew.-% an herbizidem Wirkstoff und Antidot, vorzugsweise zwischen 0,5 und 90 Gew.-%. Die Aufwandmengen an herbizidem Wirkstoff betragen 0,001 bis 0,5 kg/ha.

Der herbizide Wirkstoff V.2 wird als kommerziell formuliertes Produkt (184 g/l EC) auch allein jeweils unter zugabe von derjenigen Menge an Lösungsmittelsystem XXII (Leerformulierung) in die Spritzbrühe eingesetzt, mit welcher die antidotisch wirkende Verbindung in den in den Tabellen angegebenen Aufwandmengen ausgebracht werden.

Sämtliche antidotisch wirkende Verbindungen werden in einem Gemisch, bestehend aus 80 % Cyclohexenonen und 20 % Emulphor EL (Versuchsformulierung XXII), mit 10 % Gew.-% Wirkstoff aufbereitet.

Die Versuchsgefäße werden im Gewächshaus aufgestellt, wobei für wärmeliebende Arten 18 bis 35°C und für solche gemäßigtere Klimate 10 bis 25°C bevorzugt werden.

Die Versuchsperiode erstreckte sich über 3 bis 5 Wochen. Während dieser Zeit wurden die Pflanzen gepflegt, und ihre Reaktion auf die einzelnen Behandlungen wurde erfaßt. Bewertet wurde die Schädigung durch die chemischen Mittel anhand einer Skala von 0 % bis 100 % im Vergleich zu den unbehandelten Kontrollpflanzen. Dabei bedeutet 0 keine Schädigung und 100 eine völlige Zerstörung der Pflanzen (Triticum aestivum, Weizen).

Die anschließende Tabelle dokumentiert die antidotische Wirkung der Beispielverbindungen Nr. 1.038 und 1.039, wobei die Kulturpflanzenverträglichkeit des Cyclohexenonderivats V.2 erheblich verbessert wird.

Tabelle A

| Verringerung der durch das Herbizid V.2 verursachten Schäden an Weizen durch Kombination mit den erfindungsgemäßen Verbindungen 1.038 und 1.039 | | | | |
|---|---|---|---|---|
| Aufwandmenge Herbizider Wirkstoff | | Antidotische Verbindung | | Schädigung bei Weizen in % |
| Nr. | [kg/ha] | Nr. | [kg/ha] | |
| V.2 | 0,015 | | - | 44 |
| | 0,03 | | - | 80 |
| V.2 | 0,015 | 1.038 | 0,06 | 0 |
| | 0,03 | | 0,125 | 20 |
| V.2 | 0,015 | 1.039 | 0,06 | 0 |
| | 0,03 | | 0,125 | 10 |

**Ansprüche**

1. 1-Aryl- und 1-Hetarylimidazolcarbonsäureester der allgemeinen Formeln Ia und Ib

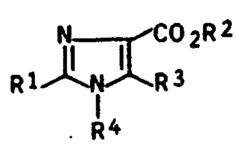

Ia

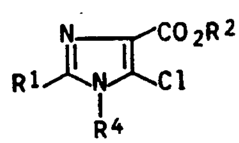

Ib

in denen die Substituenten folgende Bedeutung haben:
$R^1$ ein Halogenatom oder eine $C_1$-$C_4$-Alkylgruppe, welche ein bis drei Chloratome tragen kann,
$R^2$ eine $C_1$-$C_6$-Alkylgruppe, welche ein bis drei der folgenden Reste tragen kann: Halogen, $C_1$-$C_4$-Alkoxy und/oder $C_1$-$C_4$-Alkoxy-$C_1$-$C_4$-alkoxy, eine $C_3$-$C_6$-Alkenylgruppe, eine $C_3$-$C_6$-Alkinylgruppe oder eine $C_3$-$C_6$-Cycloalkylgruppe,
$R^3$ Wasserstoff oder eine $C_1$-$C_4$-Alkylgruppe,
$R^4$ ein Phenylring oder ein 5- oder 6-gliedriger Heteroaromat, enthaltend ein oder zwei Stickstoffatome und/oder ein Sauerstoff oder ein Schwefelatom im Ring, wobei diese aromatischen Ringe ein bis drei der folgenden Gruppen tragen können: Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, $C_1$-$C_4$-Alkylsulfinyl, $C_1C_4$-Alkylsulfonyl, $C_1$-$C_4$-Alkylcarbonyl, $C_1$-$C_4$-Alkoxycarbonyl, Carboxyl, Nitro und/oder Cyano.

2. Verfahren zur Herstellung der Verbindungen Ia gemäß Anspruch 1, dadurch gekennzeichnet, daß man einen Imidazolcarbonsäureester der Formel II

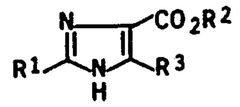
II

in an sich bekannter Weise in einem inerten organischen Lösungsmittel in Gegenwart einer Base mit einem Aryl- oder Hetarylderivat III,
$R^4$-X III
worin X ein Halogenatom bedeutzet, zu Ia umsetzt.

3. Verfahren zur Herstellung der Verbindungen Ib gemäß Anspruch 1, dadurch gekennzeichnet, daß man einen 1-Aryl- oder 1-Hetarylimidazolcarbonsäureester Ia, in dem $R^3$ Wasserstoff bedeutet,

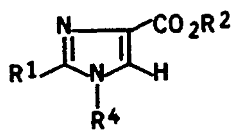
Ia ($R^3$ = H)

in an sich bekannter Weise mit einem üblichen Chlorierungsmittel zu Ib umsetzt.

4. Herbizide Mittel, enthaltend mindestens einen 1-Aryl- oder 1-Hetarylimidazolcarbonsäureester Ia und/oder Ib gemäß Anspruch 1, sowie mindestens einen herbiziden Wirkstoff aus der Gruppe

a) der 2-(4-Heteroaryloxy)- oder 2-(4-Aryloxy)-phenoxyessigsäurederivate der Formel IV,

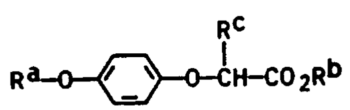
IV

in der die Substituenten folgende Bedeutung haben:
$R^a$ eine Phenylgruppe, eine Pyridylgruppe, eine Benzoxazylgruppe, eine Benzthiazylgruppe oder eine Benzpyrazinylgruppe, wobei diese aromatischen Ringsysteme bis zu zwei der folgenden Reste tragen können: Halogen, $C_1$-$C_4$-Alkyl, $C_1$-$C_4$-Halogenalkyl und/oder $C_1$-$C_4$-Halogenalkoxy
$R^b$ Wasserstoff, eine $C_1$-$C_5$-Alkylgruppe, eine $C_3$-$C_5$-Alkylideniminogruppe, eine $C_3$-$C_5$-Alkylideniminooxy-$C_2$-$C_3$-alkylgruppe oder das Äquivalent eines pflanzenverträglichen Kations und
$R^c$ Wasserstoff oder eine Methylgruppe

b) der Cyclohexenonderivate der Formel V,

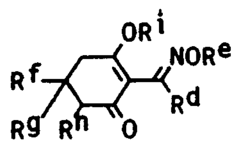
V

in welcher die Substituenten folgende Bedeutung haben:

$R^d$ eine $C_1$-$C_4$-Alkylgruppe;
$R^e$ eine $C_1$-$C_4$-Alkylgruppe, eine $C_3$-$C_4$-Alkenylgruppe, eine $C_3$-$C_4$-Alkinylgruppe, eine $C_3$-$C_4$-Halogenalkenylgruppe oder eine Thenylgruppe, welche durch ein Halogenatom substituiert sein kann;
$R^f$ eine $C_1$-$C_4$-Alkylgruppe, welche einfach durch $C_1$-$C_4$-Alkylthio oder $C_1$-$C_4$-Alkoxy substituiert sein kann; ein 5- oder 6-gliedriges gesättigtes oder einfach ungesättigtes Ringsystem, welches neben Kohlenstoffgliedern ein Sauerstoff-, ein Schwefelatom oder eine Sulfoxid- oder Sulfongruppe enthalten kann, wobei dieser Ring bis zu drei der folgenden Reste tragen kann: Hydroxy, Halogen, $C_1$-$C_4$-Alkyl, $C_1$-$C_4$-Halogenalkyl, $C_1$-$C_4$-Alkoxy und/oder $C_1$-$C_4$-Alkylthio;
einen 10-gliedrigen gesättigten oder einfach ungesättigten Heterocyclus, welcher zwei Sauerstoffatome oder Schwefelatome enthält und durch bis zu drei $C_1$-$C_4$-Alkylgruppen und/oder Methoxygruppen substituiert sein kann;
eine Phenylgruppe, eine Pyridylgruppe oder eine Isoxazolylgruppe, wobei diese Gruppen bis zu drei der folgenden Gruppen tragen können: $C_1$-$C_4$-Alkyl, $C_1$-$C_4$-Halogenalkyl, $C_1$-$C_4$-Alkoxy, $C_1$-$C_4$-Alkylthio, $C_3$-$C_6$-Alkenyloxy, $C_3$-$C_6$-Alkinyloxy, $C_1$-$C_4$-Alkoxy-$C_1$-$C_3$-alkyl, $C_1$-$C_4$-Dialkoxy-$C_1$-$C_3$-alkyl, Formyl, Halogen und/oder Benzoylamino;
$R^g$ Wasserstoff, Hy-oxy oder, wenn $R^f$ die Bedeutung $C_1$-$C_6$-Alkylgruppe hat, eine $C_1$-$C_6$-Alkylgruppe;
$R^h$ Wasserstoff, eine Cyanogruppe, ein Halogenatom oder eine $C_1$-$C_4$-Alkoxycarbonylgruppe und
$R^i$ Wasserstoff oder das Äquivalent eines umweltverträglichen Kations.

5. Herbizide Mittel nach Anspruch 4, enthaltend ein Aryl- oder Hetarylimidazolcarbonsäurederivat Ia und/oder Ib gemäß Anspruch 1 und ein Herbizid a oder ein Herbizid b), wobei das Anteilsverhältnis 4:1 bis 0,01:1 Gew.-Teile beträgt.

6. Verfahren zur selektiven Bekämpfung von unerwünschtem Pflanzenwuchs, dadurch gekennzeichnet, daß man ein Aryl- oder Hetarylimidazolcarbonsäurederivat der Formel Ia und/oder Ib gemäß Anspruch 1 und ein 2-(4-Heteroaryloxy)- oder 2-(4-Aryloxy)-phenoxyessigsäurederivat der Formel IV gemäß Anspruch 4 oder ein Cyclohexenonderivat der Formel V gemäß Anspruch 4 vor, bei oder nach der Aussaat der Kulturpflanzen, vor oder während des Auflaufens der Kulturpflanzen gleichzeitig oder nacheinander ausbringt.

7. Verfahren zur Verhinderung von Schädigungen von Kulturpflanzen durch herbizide 2-(4-Heteroaryloxy)- oder 2-(4-Aryloxy)-phenoxyessigsäure derivate der Formel IV gemäß Anspruch 4 durch herbizide Cyclohexenonderivate der Formel V gemäß Anspruch 4, dadurch gekennzeichnet, daß man das Saatgut der Kulturpflanzen mit einer antagonistisch wirksamen Menge eines Aryl- oder Hetarylimidazolcarbonsäurederivats der Formel Ia und/oder Ib gemäß Anspruch 1 behandelt.

8. Verfahren zur selektiven Bekämpfung von unerwünschtem Pflanzenwuchs, dadurch gekennzeichnet, daß man die Blätter der Kulturpflanzen und der unerwünschten Pflanzen im Nachauflaufverfahren mit einem Aryl- oder Hetarylimidazolcarbonsäurederivat der Formel Ia und/oder Ib gemäß Anspruch 1 und mit einem 2-(4-Heteroaryloxy)- oder 2-(4-Aryloxy)-phenoxyessigsäurederivat der Formel IV gemäß Anspruch 4 oder mit einem Cyclohexenonderivat der Formel V gemäß Anspruch 4 gleichzeitig oder nacheinander behandelt.

9. Verfahren gemäß den Ansprüchen 6 bis 8, dadurch gekennzeichnet, daß die Kulturpflanzen Gerste, Weizen, Mais, Kultursorghum und Reis sind.